(19) Europäisches Patentamt
European Patent Office
Office européen des brevets

(11) **EP 0 942 898 B1**

(12) **EUROPÄISCHE PATENTSCHRIFT**

(45) Veröffentlichungstag und Bekanntmachung des Hinweises auf die Patenterteilung:
**28.08.2002 Patentblatt 2002/35**

(51) Int Cl.⁷: **C07C 51/58**, B01J 19/12, C07C 53/48, C07C 53/50

(21) Anmeldenummer: **97952004.6**

(22) Anmeldetag: **28.11.1997**

(86) Internationale Anmeldenummer:
**PCT/EP97/06647**

(87) Internationale Veröffentlichungsnummer:
**WO 98/024750 (11.06.1998 Gazette 1998/23)**

(54) **HERSTELLUNG VON TRIFLUOROACETYLFLUORID**

PRODUCTION OF TRIFLUOROACETYLFLUORIDE

PREPARATION DE FLUORURE DE TRIFLUOROACETYLE

(84) Benannte Vertragsstaaten:
**DE ES FR GB IT**

(30) Priorität: **04.12.1996 DE 19650212**

(43) Veröffentlichungstag der Anmeldung:
**22.09.1999 Patentblatt 1999/38**

(60) Teilanmeldung:
**01130562.0 / 1 190 764**

(73) Patentinhaber: **Solvay Fluor und Derivate GmbH**
**30173 Hannover (DE)**

(72) Erfinder:
• **BRAUN, Max**
  **D-30900 Wedemark (DE)**
• **EICHHOLZ, Kerstin**
  **D-30479 Hannover (DE)**
• **RUDOLPH, Werner**
  **D-30559 Hannover (DE)**

(74) Vertreter: **Gosmann, Martin, Dr. et al**
**Solvay Pharmaceuticals GmbH,**
**Patentabteilung**
**Postfach 220**
**30002 Hannover (DE)**

(56) Entgegenhaltungen:
EP-A- 0 668 261          US-A- 2 736 695
US-A- 3 883 407          US-A- 5 545 298
US-A- 5 569 782

**Beschreibung**

Die Erfindung bezieht sich auf ein Verfahren zur Herstellung von Trifluoracetylfluorid durch photochemische Oxidation.

Polyhalogenierte Carbonsäurefluoride sind Zwischenprodukte in der chemischen Synthese. Sie können beispielsweise für die Herstellung wasserabstoßender bzw. ölabstoßender Verbindungen zur Anwendung bei Papier und Geweben eingesetzt werden. Sie können auch als Vorstufe für die Herstellung von oberflächenaktiven Mitteln oder im Flammschutz verwendet werden.

Die Herstellung von Perfluorcarbonsäurefluoriden kann gemäß der europäischen Patentanmeldung EP-A 260713 durch thermische Behandlung von Perfluoralkylvinylethern in Anwesenheit spezieller fluoridhaltiger Salze wie Alkalifluoride, Erdalkalifluoride, Fluormetallate, Übergangsmetallfluoriden oder Oxyfluoriden hergestellt werden. Für die thermische Behandlung ist eine Mindesttemperatur von 250 °C notwendig. Umwandlungsrate und Selektivität sind über den zeitlichen Verlauf der Reaktionsdurchführung nicht konstant: anfangs sind die Umwandlungsrate und die Selektivität gering.

Die internationale Patentanmeldung WO 96/29298 mit dem Titel "Verfahren zur Herstellung von Polyfluoracylzubereitungen" offenbart, daß man Polyhaloacylfluoride wie Trifluoracetylfluorid und Difluoracetylfluorid durch Oxidation von 1-Chloro-1,2,2,2-tetrafluorethan bzw. 1,1-Dichlor-2,2-difluorethan herstellen kann. Es handelt sich um ein thermischen Verfahren, das in bezug auf Temperatur und Druck bei oder über dem kritischen Punkt durchgeführt wird. Das Verfahren ist bezüglich der Bildung des Carbonsäurefluorids nicht sehr selektiv: bei der Herstellung von Trifluoracylverbindungen entsteht Trifluoracetylfluorid in einer Menge von mindestens 60 bis 70 Mol-%, Trifluoracetylchlorid in einer Menge von 5 bis 15 Mol-%, Trifluoressigsäure in einer Menge von 10 bis 20 Mol-%, 5 bis 10 Mol-% eines Dimerisationsproduktes (Octafluordichlorbutan), und 1 bis 5 Mol-% Chlorfluorphosgen.

Aufgabe der vorliegenden Erfindung ist es, ein Verfahren anzugeben, mit welchem auf technisch einfache Weise bei hohem Umsatz und hoher Selektivität Trifluoracetylfluorid hergestellt werden können. Diese Aufgabe wird durch das erfindungsgemäße Verfahren gelöst.

Das erfindungsgemäße Verfahren zur Herstellung von Trifluoracetylfluorid umfaßt die photochemische Oxidation von $CF_3CHClF$ mit Sauerstoff als Reaktant in der Gasphase. Man arbeitet also unter aktivierender Bestrahlung der gasförmigen Reaktionsmischung. Dabei kann die Bestrahlung durch Glas beliebiger Zusammensetzung (beispielsweise durch Glas innerhalb der Apparaturen, Glaseinfassungen des Strahlers) erfolgen. Beispielsweise kann man Quarzglasapparaturen einsetzen. Die Umsetzungsrate wird erhöht, wenn man in Anwesenheit eines Sensibilisators, insbesondere in Anwesenheit von elementarem Chlor als Sensibilisator, arbeitet. Dabei kann man Strahler wie Bestrahlungslampen und Leuchtstoffröhren einsetzen, die beispielsweise innerhalb eines Bereichs von 200 bis 600 nm abstrahlen. Vorteilhaft nimmt man eine aktivierende Bestrahlung mit Licht einer Wellenlänge von $\lambda \geq 280$ nm vor. Anhand dieser bevorzugten Ausführungsform wird die Erfindung weiter erläutert.

Umsatzrate, Ausbeute und Selektivität sind besonders hoch, wenn man in Anwesenheit von elementarem Chlor umsetzt und eine aktivierende Bestrahlung mit Licht einer Wellenlänge $\lambda \geq 280$ nm vornimmt. Frequenzen einer Wellenlänge unterhalb von 280 nm sind dann im wesentlichen aus dem Frequenzspektrum ausgeblendet. Dies kann man dadurch bewirken, daß man Bestrahlungslampen verwendet, die nur Licht einer Wellenlänge oberhalb oder bei 280 nm abstrahlen, und/oder man verwendet Mittel, die die entsprechenden Frequenzen aus dem abgestrahlten Licht ausblenden. Beispielsweise kann man durch Glas bestrahlen, welches nur für Licht einer Wellenlänge von 280 nm oder darüber durchlässig ist, also den kürzerwelligeren Strahlungsanteil herausfiltert. Gut geeignet dafür sind beispielsweise Borosilikat-Gläser. Geeignete Gläser enthalten beispielsweise 7 bis 13% $B_2O_3$, 70 bis 80% $SiO_2$, ferner 2 bis 7% $Al_2O_3$ und 4 bis 8% $Na_2O + K_2O$ sowie 0 bis 5% Erdalkalimetalloxide (jeweils Gew.-%). Bekannte Warenzeichen für Borosilikat-Gläser sind Duran, Pyrex und Solidex.

Zur Bestrahlung sind besonders gut Bestrahlungslampen geeignet, die nur (UV)Licht einer Wellenlänge oberhalb von oder bei 280 nm abstrahlen. Insbesondere Leuchtstoff-Röhren, (z.B. von der Firma Philips) sind sehr gut geeignet. Mit derartigen Lampen kann man die Bestrahlung durch Quarzglas, aber auch durch die vorstehend beschriebenen, den kürzerwelligen Bestrahlungsanteil herausfilternde Gläser vornehmen. Voraussetzung ist natürlich, daß die verwendeten Lampen oder Röhren auch im Absorptionsbereich des elementaren Chlors emittieren. Neben den besonders gut geeigneten Leuchtstoffröhren kann man auch beispielsweise Bestrahlungslampen (z.B. Quecksilber-Mittel-oder Hochdruckstrahler) verwenden; etwaige Linien im Bereich unterhalb von 280 nm werden herausgefiltert, beispielsweise indem man durch ein Glas bestrahlt, daß nur für Licht einer Wellenlänge bei oder oberhalb von 280 nm durchlässig ist. Verwendbare Gläser sind weiter oben beschrieben. Gut geeignet zur Bestrahlung sind auch Lampen, z.B. Quecksilber-Hochdrucklampen, die aufgrund eines Dotierungsmittels überwiegend oder nur im bevorzugten Wellenbereich bei oder oberhalb von 280 nm abstrahlen. Quecksilber-Hochdruckstrahler beispielsweise weisen eine recht intensive Bande im Bereich von 254 nm auf, die, wie oben beschrieben wird, beispielsweise durch Borosilikat-Glas herausgefiltert werden kann. Bei durch Metalljodide dotierten Quecksilber-Hochdruckstrahlern ist diese Linie stark unterdrückt. Überraschend ist die oft überproportionale Erhöhung der Umsatzrate bei Verwendung solcher dotierter Strahler. Besonders gut geeignet sind Quecksilber-Hochdruckstrahler, die mit Galliumjodid, insbesondere Thalliumjodid oder Cadmiumjodid dotiert sind. Auch bei Verwendung solcher dotierter Strahlungslampen filtert man vorteilhaft den kürzerwelligen Strahlungsanteil mit λ < 280 nm heraus, beispielsweise indem man in Borosilikat-Glas arbeitet.

In Bezug auf Umsetzungstemperatur und Druck kann man derart arbeiten, daß es zu keiner Kondensation innerhalb des Photoreaktors kommt. Bei Einsatz höhersiedender Edukte kann man beispielsweise im Vakuum arbeiten. In Bezug auf die Temperatur arbeitet man vorteilhaft bei Temperaturen bis zu 200°C. Bevorzugter Temperaturbereich ist 30 bis 150°C. Wie gesagt kann man bei vermindertem Druck arbeiten, vorzugsweise beträgt der Druck mindestens 1 bar absolut. Besonders bevorzugt arbeitet man bei einem Druck von 1 bis 10 bar (abs.). Ganz besonders bevorzugt arbeitet man drucklos. Der Begriff "drucklos" bedeutet im Rahmen der vorliegenden Erfindung, daß auf die Reaktionsmischung außer dem Umgebungsdruck (d.h. etwa 1 bar), dem Förderdruck des Sauerstoffgases (bzw. des sauerstoffhaltigen Gases, man kann z.B. Luft oder Sauerstoff-/Inertgas-Gemische einsetzen) und das gegebenenfalls eingesetzten Chlors sowie dem sich gegebenenfalls ausbildenden Druck durch bei der Reaktion entstehendes Chlorwasserstoffgas kein zusätzlicher Druck einwirkt. Der Gesamtdruck im Reaktor ist dann zweckmäßig kleiner als 2 bar absolut, je nach Förderdruck sogar kleiner als 1,5 bar absolut, aber größer als der Umgebungsdruck.

Das Verfahren kann batchweise oder kontinuierlich durchgeführt werden, wobei man die Umsetzung vorteilhaft in einer Durchflußapparatur durchführt. Vorzugsweise geht man so vor, daß man kontinuierlich Ausgangsmaterial (das entsprechende Edukt, Sauerstoff bzw. ein sauerstoffenthaltendes Gas und gegebenenfalls Chlor) in die Durchflußappatur einspeist und entsprechend der eingespeisten Menge kontinuierlich Reaktionsprodukt abzieht.

Das Molverhältnis zwischen dem Edukt und Sauerstoff kann in einem weiten Bereich schwanken, zweckmäßig setzt man jedoch mindestens 0,4 Mol Sauerstoff pro Mol Ausgangsverbindung ein. Besonders gute Ergebnisse werden erzielt, wenn das Molverhältnis zwischen der Ausgangsverbindung und dem Sauerstoff im Bereich von 1:0,4 bis 1:1, insbesondere 1:0,4 bis 1:0,6 liegt. Der Sauerstoff kann wie gesagt in Form von Luft, als $O_2$/Inertgas-Gemisch, vorzugsweise aber als reiner Sauerstoff eingesetzt werden. Sofern man die Bestrahlung in Anwesenheit von Chlor als Sensibilisator vornimmt, kann das Molverhältnis zwischen dem Edukt und dem elementarem Chlor ebenfalls in einem weiten Bereich schwanken z.B. 1:0,01 bis 1:1. Besonders gut Ergebnisse werden erzielt, wenn das Molverhältnis zwischen dem Edukt und dem elementarem Chlor im Bereich von 1:0,05 bis 1:0,2 liegt.

In Bezug auf die Produktreinheit ist es wünschenswert, daß möglichst wenig Wasser bei der Umsetzung vorhanden ist (beispielsweise weniger als 30 ppm). Gewünschtenfalls kann man die Reaktanten in bekannter Weise von mitgeschlepptem Wasser befreien, z.B. mittels Molekularsieb.

Die durchschnittliche Verweilzeit im Reaktionsgefäß liegt vorteilhaft zwischen 0,01 und 30 Minuten, vorzugsweise zwischen 0,5 und 3,5 Minuten. Die optimale durchschnittliche Verweilzeit, die u.a. von der Art der Lampen, der Strahlungsleistung der Lampen und von geometrischen Parametern der Bestrahlungsapparatur abhängig ist, kann man durch einfache Handversuche und Analyse des Produktstroms, beispielsweise durch Gaschromatographie, ermitteln. Vorteilhaft kann es auch sein, die Reaktionsmischung beispielsweise durch geeignete Einbauten im Reaktor gut zu verwirbeln. Der Reaktionsmischung braucht kein flüssiges Kühlmittel beigefügt zu werden.

Man stelltfolgendes Carbonsäurefluorid :

$$CF_3CHClF + 1/2\ O_2 \rightarrow CF_3C(O)F + HCl$$

Zum Schutz der Oberfläche der Glasapparaturen kann man diese modifizieren, um sie gegen den Angriff durch das enstehende HCl-Gas und etwaig spurenmäßig entstehenden HF zu schützen. Hierzu kann man beispielsweise transparente Folien oder "Strümpfe" über die zu schützenden Flächen ziehen. Als Material eignen sich poly- oder perfluorierte Kunststoffe wie Polytetrafluorethylen (PTFE), Perfluorpolypropylen (FEP), Perfluoralkoxy-Polymere (PFA) oder Polyvinylidendifluorid (PVDF) und ihre Gemische.

Das Verfahren hat gegenüber dem bekannten Verfahren zur Herstellung von Carbonsäurefluoriden den Vorteil, daß es mit hohem Umsatz und hoher Selektivität bei niedrigen Temperaturen durchgeführt werden kann. Es ist deshalb sehr effektiv. Außerdem kann es gewünschtenfalls bei niedrigem Druck durchgeführt werden.

Eine Bestrahlungsapparatur gemäß nachfolgenden Beschreibung ist im erfindungsgemäßen Verfahren verwendbar und ist speziell modifiziert. Bestrahlungsapparaturen weisen gewöhnlich einen Behälter zur Aufnahme einer zu bestrahlenden Flüssigkeit, eines Gases oder Gasgemisches auf sowie ein Behältnis, in welchem der Strahler angeordnet ist. Häufig weisen solche Apparate einen Schacht auf, in welchem der Strahler angeordnet ist (Tauchschachtreaktoren). Bekannt sind Bestrahlungsapparaturen aus Glas, z.B. Quarz- oder Borosilikatglas mit Glasbehältnis. Die Bestrahlungsapparatur weist einen Behälter zur Aufnahme eines zu bestrahlenden Gases und einen Strahler auf, welcher in einem Behältnis angeordnet ist, das in Kontakt mit dem zu bestrahlenden Gas steht, wobei mindestens ein Teil der in Kontakt mit dem zu bestrahlenden Gas stehenden Oberfläche des Behältnisses eine strahlungs-durchlässige Schutzbeschichtung auf Basis von strahlungsdurchlässigen polyfluorierten oder perfluorierten Polymeren mindestens auf einen Teil der in Kontakt mit dem zu bestrahlenden Gas stehenden Oberfläche des Behältnisses aufweist, wobei die Schutzbeschichtung ein Schrumpfschlauch ist, der auf dem Glasbehältnis des Strahlers aufgezogen ist. Alternativ, aber nicht erfindungsgemäß kann der Tauchschacht, das Behältnis oder die gesamte Apparatur aus strahlungsdurchlässigem (d.h. lichtdurchlässigem) Polymer gebildet sein, vorzugsweise aus polyfluoriertem oder perfluoriertem Polymer wie PTFE, FEP, PFA, PVDF oder deren Gemische. Bevorzugte Schutzbeschichtungen sind oben genannt, z.B. solche aus Polytetrafluorethylen (PTFE), FEP oder deren Gemische. Besonders bevorzugt sind Bestrahlungsapparaturen, welche eine Schutzbeschichtung in Form eines PTFE- oder PTFE-FEP-Schrumpfschlauches aufweisen, der auf dem Glasbehältnis des Strahlers aufgezogen ist. Insbesondere handelt es sich bei den Bestrahlungsapparaturen um Tauchschachtreaktoren, wobei die Schutzbeschichtung (bevorzugt ein PTFEoder TTFE-FEP-Schrumpfschlauch) auf dem Kühlfinger des Tauchschachtes aufgebracht ist, d.h. auf derjenigen Glasoberfläche des Kühlwassermantels, welche in Kontakt mit dem zu bestrahlenden Gas oder Gasgemisch steht. Geeignete Polymere, die bei der Anwendungstemperatur klar sind und deshalb lichtdurchlässig, sind im Handel erhältlich.

Die folgenden Beispiele sollen die Erfindung weiter erläutern, ohne sie in ihrem Umfang einzuschränken. Man arbeitet unter Beachtung der für das Arbeiten mit molekularem Sauerstoff notwendigen Sicherheitsvorkehrungen. Bei der Verwendung von PTFE- oder anderen Beschichtungen kann es zu statischen Aufladungen kommen, die durch eine Erdung, beispielsweise ein Metallnetz, abgeleitet werden können.

Beispiele

Beispiele 1 bis 4:

Herstellung von Trifluoracetylfluorid aus 2-Chlor-1,1,1,2-tetrafluorethan (124) mit Hg-Tauchstrahlern

Lampen: handelsübliche Tauchstrahler der Firma Heraeus Noblelight

verwendeter Tauchstrahler:

| Beispiel 1 | TQ 718 nicht dotiert |
|---|---|
| Beispiel 2 | TQ 718 mit Cadmiumjodid dotiert |
| Beispiel 3 | TQ 718 nicht dotiert |
| Beispiel 4 | TQ 718 nicht dotiert |

Aufbau und Durchführung

Beispiel 1: nicht dotierter Strahler, Borosilikat-Glas

In einen ca. 900 ml fassenden Tauchschacht-Photoreaktor mit Kühlfingereinsatz für Tauchlampen aus Duran 50$^R$(Wasserkühlung) wurden 101,3 g (0,74 mol) 124, 15,1 g (0,47 mol) Sauerstoff und 5,2 g (0,07 mol) Chlor eingespeist. Alle 3 Substanzen wurden vor dem Reaktor gemischt und gemeinsam gasförmig eingeleitet. Vor dem Versuch ist die Tauchlampe 15 Minuten vorgeglüht worden, da sie erst nach dieser Zeit ihre volle Strahlungsintensität erreicht. Während dieser Zeit wurde der Reaktor mit Stickstoff gespült. Die Temperatur betrug während der 30-minütigen Reaktion 80°C. In diesem Versuch wurde ein Umsatz von 85,46 % erzielt, mit einer Selektivität bezogen auf entstandenes Trifluoracetylfluorid von 99,90%. Im Abgas wurden Spuren von TFAC nachgewiesen.

Analytik

Das Reaktorabgas wurde mittels GC untersucht, die Identifikation der Substanzen erfolgte mit GC-MS.

Beispiel 2: CdI-dotierter Strahler, Borosilikat-Glas

| Durchführung | wie Beispiel 1 |
|---|---|
| Ansatz | 105,4 g (0,77 mol) 124, 14,4 g (0,45 mol) Sauerstoff, 5,1 g (0,07 mol) Chlor |
| Reaktions- temperatur | 90°C |

(fortgesetzt)

| Umsatz | 93,09% |
| Selektivität | 98,03% |

Aufbau und Durchführung

Beispiel 3: nicht dotierter Strahler, Quarz-Glas

In einen ca. 900 ml fassenden Tauchschacht-Photoreaktor mit Kühlfingereinsatz für Tauchlampen aus Quarz (Wasserkühlung) wurden 69,2 g (0,51 mol) 124, 10,0 g (0,31 mol) Sauerstoff und 3,2 g (0,05 mol) Chlor eingespeist. Alle 3 Substanzen wurden vor dem Reaktor gemischt und gemeinsam gasförmig eingeleitet. Vor dem Versuch ist die Tauchlampe 15 Minuten vorgeglüht worden, da sie erst nach dieser Zeit ihre volle Strahlungsintensität erreicht. Während dieser Zeit wurde der Reaktor mit Stickstoff gespült. Die Temperatur betrug während der 20-minütigen Reaktion 115°C. In diesem Versuch wurde ein Umsatz von 97,75% erzielt, mit einer Selektivität bezogen auf entstandenes Trifluoracetylfluorid von 91,38%. Im Abgas wurden Spuren von Trifluoracetylchlorid nachgewiesen.

Beispiel 4: nicht dotierter Strahler, Quarzglas, chlorfrei

| Durchführung | wie Beispiel 3, Tauchschacht-Photoreaktor mit Kühlfingereinsatz aus Quarz |
| Ansatz | 109,8 g (0,80 mol) 124, 15,2 g (0,48 mol) Sauerstoff ohne Chlor |
| Reaktionstemperatur | 135°C |
| Umsatz | 92,13% |
| Selektivität | 90,85% |

Beispiel 5:

Herstellung von Trifluoracetylfluroid aus 2-Chlor-1,1,1,2-tetrafluorethan mit Leuchtstofflampen als Strahlungsquelle

| Ansatz | 118,07 g 124 = 0,91 mol | |
| | 19,84 g $O_2$ = 0,62 mol | |
| | 9,93 g $Cl_2$ = 0,14 mol | |
| Lampen | 3 x 40 Watt UV Lampen von Philips | |
| | Typ | Cleo Performance R-UVA 40 W |
| | Länge | 600 mm |

Aufbau und Durchführung

In einen 4,3 Liter fassenden Photoreaktor ( Ø 100 mm, Wandstärke 2 mm) aus Duran 50 wurde eine Mischung aus 118,07 g (0,91 mol) 124, 19,84 g (0,62 mol) Sauerstoff und 9,93 g (0,14 mol) Chlor gasförmig eingeleitet.
Die Reaktortemperatur betrug während der 30-minütigen Reaktion 41 bis 46°C. Die Bestrahlung erfolgte mit 3 handelsüblichen UV Leuchtstofflampen der Firma Philips, die einseitig mit einer Reflektorschicht ausgestattet sind, um die Strahlungsausbeute zu erhöhen (Anordnung der Lampen zylindrisch um den Reaktor).
Der Umsatz der Reaktion betrug 65,17%, die Selektivität, bezogen auf entstandenes TFAF, lag bei 99,88%. Im GC konnten ebenfalls Spuren von Trifluoracetylchlorid nachgewiesen werden.

Analytik

Die Probenanalyse des Reaktorabgases erfolgte mittels GC, die Identifikation der Substanzen mittels GC-MS.

Beispiel 6 (Vergleichsbeispiel):

Herstellung von Chlordifluoracetylfluorid aus 1,2-Dichlor-1,1,2-trifluorethan (123a) in Duranglas

Ansatz  80,10 g 123a = 0,53 mol
10,60 g $O_2$ = 0,33 mol
3,90 g $Cl_2$ = 0,06 mol

Aufbau und Durchführung

Durch einen 4,3 Liter fassenden Photoreaktor aus Duran 50 (Reaktoraufbau wie in Beispiel 5) werden Sauerstoff, Chlor und 123a geleitet. Sauerstoff und Chlor werden vorgemischt und nach dem auf 100°C temperierten Vorverdampfer dem 123a zugeführt. Die Dosierung des 123a in den Vorverdampfer erfolgt über eine Membranpumpe mit kühlbarem Kopf.

Die Bestrahlung erfolgt mit handelsüblichen UV Leuchtstofflampen der Firma Philips, die einseitig mit einer Reflektorschicht ausgestattet ist, um die Strahlungsausbeute zu erhöhen. Die genaue Bezeichnung ist Cleo Performance R-UVA 40 W. Die Nennleistung der Lampen beträgt 40 Watt.

Versuchsdauer 15 Minuten

123a wird verdampft und mit den anderen Reaktionskomponenten gemischt durch den Reaktor geleitet. Hinter dem Reaktor befinden sich zwei Kühlfallen mit einer Methanol/$CO_2$-Mischung, um das entstandene Chlordifluoracetylfluorid aufzufangen.

Die Reaktortemperatur betrug dabei bis zu 46°C, bestrahlt wurde mit 2 x 40 Watt.

Der Umsatz betrug 30,66%, die Selektivität bezogen auf entstandenes CDFAF lag bei 93,41%.

Analytik

Die Proben wurden mittels Gaschromatographie untersucht, die Identifikation der Substanzen erfolgte mittels GC-MS.

Beispiel 7:

Herstellung von Trifluoracetylfluorid aus R 124 mit modifiziertem Reaktor

Apparatur:

Eingesetzt wurde ein Photoreaktor mit 900 ml Reaktorvolumen. Er weist einen Tauchschacht mit wassergekühltem Kühlfinger zur Aufnahme des Strahlers auf. Der Kühlfinger des Tauchschachtes aus Duran 50 Glas war auf der in Kontakt mit dem zu bestrahlenden Gas stehenden Oberfläche mit einem Schrumpfschlauch TEFLON-FEP[R] der Firma Technoplast überzogen. eingesetzte Lampe hatte eine Leistung von 700 W (TQ 718, Heraeus nicht dotierter Strahler).

Durchführung:

Eine Mischung von 51,3 g (0,37 mol) 124, 6 g (0,19 mol) Sauerstoff und ca. 2,5 g (0,03 mol) Chlor wurde während einer Zeitdauer von 15 Minuten durch den Reaktor geleitet (Innentemperatur: 85°C). Eine Veränderung des Schrumpfschlauches (Trübung, Gelbfärbung) wurde nicht beobachtet. Der Umsatz betrug 94,1%, die Selektivität 99,6%.

Auch bei einem Langzeittest war keine Veränderung des Schrumpfschlauches zu beobachten.

**Patentansprüche**

1.  Verfahren zur Herstellung von $CF_3C(O)F$ durch photochemische Oxidation von $CF_3CHClF$ mit Sauerstoff als Reaktant in der Gasphase.

2.  Verfahren nach Anspruch 1, **dadurch gekennzeichnet, daß** man in Anwesenheit von elementarem Chlor als Sensibilisator arbeitet.

3. Verfahren nach Anspruch 2, **dadurch gekennzeichnet, daß** man in Anwesenheit von elementarem Chlor arbeitet und eine aktivierende Bestrahlung mit Licht einer Wellenlänge $\lambda \geq 280$ nm vornimmt.

4. Verfahren nach Anspruch 1, **dadurch gekennzeichnet, daß** man die Umsetzung in einer Durchflußapparatur durchführt.

5. Verfahren nach Anspruch 4, **dadurch gekennzeichnet, daß** man kontinuierlich Edukt, Sauerstoff und gegebenenfalls Chlor in die Durchflußapparatur einspeist und Carbonsäurefluorid kontinuierlich abzieht.

6. Verfahren nach Anspruch 1, **dadurch gekennzeichnet, daß** man bei einem Druck von 1 bis 2 bar (absolut) arbeitet.

7. Verfahren nach Anspruch 1, **dadurch gekennzeichnet, daß** das Molverhältnis zwischen dem Edukt und Sauerstoff ($O_2$) im Bereich von 1:0,1 bis 1:1,5, vorzugsweise im Bereich von 1:0,4 bis 1:0,6 liegt.

8. Verfahren nach Anspruch 2, **dadurch gekennzeichnet, daß** das Molverhältnis zwischen dem Edukt und dem elementaren Chlor im Bereich von 1:0,01 bis 1:1, vorzugsweise im Bereich von 1:0,05 bis 1:0,2 liegt.

9. Verfahren nach Anspruch 1, **dadurch gekennzeichnet, daß** man die photochemische Oxidation bei einer Temperatur von bis zu 200 °C, vorzugsweise in einem Temperaturbereich von 30 bis 90 °C durchführt.

10. Verfahren nach Anspruch 4, **dadurch gekennzeichnet, daß** die durchschnittliche Verweilzeit in der Durchflußapparatur zwischen 0,01 und 30 Minuten, vorzugsweise zwischen 0,5 und 3,5 Minuten liegt.

11. Verfahren nach Anspruch 1, **dadurch gekennzeichnet, daß** man zur aktivierenden Bestrahlung einen dotierten Strahler, vorzugsweise einen mit einem Metalljodid dotierten Hg-Hochdruckstrahler einsetzt.

12. Verfahren nach Anspruch 1, **dadurch gekennzeichnet, daß** man zur aktivierenden Bestrahlung Leuchtstoffröhren verwendet, insbesondere Leuchtstoffröhren, die nur (UV-)Licht einer Wellenlänge oberhalb oder bei 280 nm abstrahlen.

**Claims**

1. A process for the preparation of $CF_3C(O)F$ by photochemical oxidation of $CF_3CHClF$ with oxygen as reactant in the gas phase.

2. A process according to Claim 1, **characterised in that** operation is in the presence of elemental chlorine as sensitiser.

3. A process according to Claim 2, **characterised in that** operation is in the presence of elemental chlorine and activating irradiation with light of a wavelength $\lambda \geq 280$ nm is carried out.

4. A process according to Claim 1, **characterised in that** the reaction is performed in a flow apparatus.

5. A process according to Claim 4, **characterised in that** educt, oxygen and optionally chlorine are fed continuously into the flow apparatus and carboxylic acid fluoride is continuously withdrawn.

6. A process according to Claim 1, **characterised in that** operation is at a pressure of 1 to 2 bar (absolute).

7. A process according to Claim 1, **characterised in that** the molar ratio between the educt and oxygen ($O_2$) lies in the range of 1:0.1 to 1:1.5, preferably in the range of 1:0.4 to 1:0.6.

8. A process according to Claim 2, **characterised in that** the molar ratio between the educt and the elemental chlorine lies in the range of 1:0.01 to 1:1, preferably in the range of 1:0.05 to 1:0.2

9. A process according to Claim 1, **characterised in that** the photochemical oxidation is carried out at a temperature of up to 200°C, preferably in a temperature range of 30 to 90°C.

10. A process according to Claim 4, **characterised in that** the average dwell time in the flow apparatus is between 0.01 and 30 minutes, preferably between 0.5 and 3.5 minutes.

11. A process according to Claim 1, **characterised in that** a doped radiator, preferably a high-pressure Hg radiator doped with a metal iodide, is used for the activating radiation.

12. A process according to Claim 1, **characterised in that** fluorescent tubes are used for the activating irradiation, in particular fluorescent tubes which only emit (UV) light of a wavelength above or at 280 nm.

**Revendications**

1. Procédé de préparation de $CF_3C(O)F$ par oxydation photochimique de $CF_3CHClF$ avec de l'oxygène comme réactif dans la phase gazeuse.

2. Dispositif selon la revendication 1, **caractérisé en ce que** l'on travaille en présence de chlore élémentaire en tant que sensibilisateur.

3. Procédé selon la revendication 2, **caractérisé en ce que** l'on travaille en présence de chlore élémentaire et **en ce que** l'on procède à une irradiation activante avec de la lumière présentant une longueur d'onde $\lambda \geq 280$ nm.

4. Procédé selon la revendication 1, **caractérisé en ce que** l'on réalise la conversion dans un appareil à flux continu.

5. Procédé selon la revendication 4, **caractérisé en ce que** l'on injecte en continu un éduit, de l'oxygène et éventuellement du chlore dans l'appareil à flux continu, et **en ce que** l'on soutire en continu du fluorure d'acide carboxylique.

6. Procédé selon la revendication 1, **caractérisé en ce que** l'on travaille à une pression comprise entre 1 et 2 bars (absolus).

7. Procédé selon la revendication 1, **caractérisé en ce que** le rapport molaire entre Réduit et l'oxygène ($O_2$) est compris dans la gamme allant de 1:0,1 à 1:1,5, de préférence dans la gamme allant de 1:0,4 à 1:0,6.

8. Procédé selon la revendication 2, **caractérisé en ce que** le rapport molaire entre l'éduit et le chlore élémentaire est compris dans la gamme allant de 1:0,01 à 1:1, de préférence dans la gamme allant de 1:0,05 à 1:0,2.

9. Procédé selon la revendication 1, **caractérisé en ce que** l'on conduit l'oxydation photochimique à une température allant jusqu'à 200 °C, de préférence dans une gamme de températures comprise entre 30 et 90 °C.

10. Procédé selon la revendication 4, **caractérisé en ce que** le temps de séjour moyen dans l'appareil à flux continu est compris entre 0,01 et 30 minutes, de préférence entre 0,5 et 3,5 minutes.

11. Procédé selon la revendication 1, **caractérisé en ce que** l'on utilise pour réaliser l'irradiation activante un émetteur dopé, de préférence un émetteur à vapeur de mercure haute pression dopé au iodure de métal.

12. Procédé selon la revendication 1, **caractérisé en ce que** l'on utilise pour l'irradiation activante des tubes fluorescents, en particulier des tubes fluorescents qui émettent uniquement de la lumière (UV) d'une longueur d'onde supérieure ou égale à 280 nm.